# EUROPEAN PATENT SPECIFICATION

(11) **EP 4 362 786 B1**
(45) Date of publication and mention of the grant of the patent: **29.04.2026**
(21) Application number: 22733686.4
(22) Date of filing: 01.07.2022
(51) Int. Cl.: A61B 5/117, A24F 40/50

(54) **DEVICE INCORPORATING BIOACOUSTICS SENSING**
VORRICHTUNG MIT BIOAKUSTIKERFASSUNG
DISPOSITIF INCORPORANT UNE DÉTECTION BIOACOUSTIQUE

(30) Priority: 02.07.2021 EP 21183468
(43) Date of publication of application: 08.05.2024
(73) Proprietor: Philip Morris Products S.A., 2000 Neuchâtel (CH)
(72) Inventor: ELLIOTT-BOWMAN, Bernadette, 1015 Lausanne (CH); WRIGHT, Christopher John, 1015 Lausanne (CH); LAU, Wai Keith, 1015 Lausanne (CH)
(74) Representative: Grünecker Patent- und Rechtsanwälte PartG mbB
(86) International application number: PCT/EP2022/068332
(87) International publication number: WO 2023/275384

(56) References cited:
- WO-A1-2020/183476
- US-A1- 2015 122 252
- US-A1- 2021 015 162
- SIM JOO YONG ET AL: "Identity Recognition Based on Bioacoustics of Human Body", IEEE TRANSACTIONS ON CYBERNETICS, IEEE, PISCATAWAY, NJ, USA, vol. 51, no. 5, 4 October 2019 (2019-10-04), pages 2761 - 2772, XP011850084, ISSN: 2168-2267, [retrieved on 20210414], DOI: 10.1109/TCYB.2019.2941281

## Description

The present disclosure relates to systems and methods for capturing information related to a user aspect or a consumable aspect of an aerosol-generating device.

US 9430043 B1 proposes employing modulations in an acoustic signal transmitted thorough a user's hand to identify a current hand gesture. The acoustic signal may be detected at a wristband carried by the user. The wristband has a signal processing component attached thereto to generate signals for controlling an electronic device based on the identified hand gesture.

WO 2014/172905 A1 relates to identifying a user of an electronic cigarette. The user inputs user information by using an input device, wherein the user information may include biometric information such as voice information, fingerprint information and iris information, vein infrared information, facial information, and palm shape information.

WO 2020/183476 A1 relates to identification, authorization, registration and operation of a fluid delivery system for delivering substances to individuals. The disclosure provides for a method including transmitting waves towards at least a portion of a mouth of an individual, sensing, by a device, at least a portion of reflections of the waves from the individual, deriving an oral signature based on the sensing, and storing an indication of the oral signature in a database.

US 2021/015162 A1 describes an aerosol delivery device including a chamber for receiving an article including an aerosolizable material for delivery by the aerosol delivery device, a transmitter, a receiver spaced apart from the transmitter, and a processor. The processor is configured to cause the transmitter to transmit a first signal to the receiver at least partially through an article in the chamber in use, so that the receiver receives a second signal, wherein the second signal is the first signal altered by interaction with a signal altering component of the article, and determine article data from the second signal.

Sim Joo Yong et al.: "Identity Recognition Based on Bioacoustics of Human Body", IEEE TRANSACTIONS ON CYBERNETICS, vol. 51, no. 5, 4 October2019, pages 2761-2772, ISSN: 2168-2267, DOI: 10.1109/TCYB.2019.2941281 describe a bioacoustic frequency spectroscopy system applied to fingers to obtain information on anatomy, biomechanics, and biomaterial properties of tissues. As a result, modulated vibrations propagating through a human body capture a unique spectral trait of a person.

US 2015/122252 A1 discloses a vaporizer including a vaporizer control module functionally coupled to the vaporizer and configured to control function of the vaporizer. The vaporizer control module automatically restrict use of the vaporizer after a predefined script is executed. The vaporizer includes a fingerprint scanner functionally coupled to a push button and configured to scan a fingerprint. A locking module is configured to securely lock the vaporizer from use if an authorized fingerprint is not detected through the fingerprint scanner.

According to an aspect of the present invention, there is provided a system for capturing information related to an aspect associated with an aerosol-generating device, the aspect comprising at least one of a user aspect and a consumable aspect. The system comprises a signal capture system comprising one or more acoustic actuators configured for generating an acoustic signal, and an acoustic sensor. The one or more acoustic actuators and the acoustic sensor are located at predetermined locations with respect to the aerosol-generating device such that an acoustic signal generated by at least one of the one or more acoustic actuators is modulated at least in part according to the aspect before being detected by the acoustic sensor, thereby capturing the information related to the aspect, wherein the pressing of the button generates the acoustic signal from a mechanical motion of the button.

The system according to the invention hence captures information based on a challenge response mechanism wherein an acoustic signal applied to one location is detected by an acoustic sensor at a defined other location after transmission through the human body.

Acoustic signals passing the human body are modulated by unique variations in anatomy and physiology, such as joint tension, soft tissue properties and bone properties. Therefore, the captured information is a physical information very difficult to counterfeit and analyzing the modulated acoustic signal may be employed to authenticate a user may be with high accuracy.

Biometric authentication is secure because biological features are unique to each individual as a result of natural variation making it extremely rare for two individuals to share the same features. However, most commonly used biometrics, such as fingerprints and facial features, are vulnerable to being stolen and require special hardware to capture the biometric data The present invention hence provides for a solution overcoming this problem. In addition, the bioacoustic signal is obtained directly via sensor components of the aerosol-generating device instead of relying on externally obtained user information.

Alternatively, or additionally, the system captures information based on applying an acoustic signal to one location such that the acoustic signal is modulated by transmission through a consumable, and detecting the modulated acoustic signal at a defined other location.

The captured information may relate to a consumable aspect allowing authenticating the consumable. The modulations in the acoustic signature caused by a consumable are influenced by material density, material viscosity, material composition, and comparable material properties that in uniquely characterize a tobacco products or e-liquids by certified manufacturers. The claimed solution hence allows verifying authenticity of the consumable on a physical basis that is less prone to counterfeiting. Hence, the present invention improves protection of the user from exposure to potentially harmful aerosols from uncertified manufacturers , counterfeited tobacco products, or dangerous e-liquids.

The predetermined locations of the acoustic actuators and the acoustic sensor may correspond to points of contact of a user's digits on the aerosol-generating device when the user is holding the aerosol-generating device.

Hence, capturing the information of the user aspect or the consumable aspect can be carried out without burdening the user with particular actions beyond merely using the aerosol-generating device.

The one or more acoustic actuators comprises a button. The acoustic signal generated by the button may comprise a first acoustic signal generated upon an activation or operation of the button and a second acoustic signal generated upon a release of the button.

Detecting the first acoustic signal and the second acoustic signal may increase the amount of captured information.

Alternatively to the acoustic signal generated by the button comprising a first acoustic signal and a second acoustic signal, the aerosol-generating device may be configured such that an activation or operation of the button activates the signal capture system, and a release of the button generates the acoustic signal.

The acoustic sensor may be comprised in a smart watch.

The one or more acoustic actuators comprise a vibration speaker, wherein the acoustic signal comprises a series of acoustic signals generated by the vibration speaker. Advantageously, a casing of the aerosol-generating device comprises perforations at the predetermined location of the acoustic sensor.

Hence, the human body or the consumable may be probed with a series of acoustic signals, so as to provide information differential with respect to frequency.

An acoustic analysis system may be provided which is configured for assessing the aspect on basis of the detected acoustic signal, wherein the acoustic analysis system is configured for assessing the aspect by comparing the detected acoustic signal with one or more reference signals.

The aspect may comprise the user aspect, wherein the information related to the user aspect allows assessing a user of the aerosol-generating device, wherein the one or more reference signals comprise bioacoustic profiles of registered users of the aerosol-generating device, wherein the acoustic analysis system is configured for performing an identification of the user from among the registered users.

Assessing the user of the aerosol-generating device may protect the aerosol-generating device from unauthorized use.

The aerosol-generating device may be configured to, in response to identifying the user, adjust a process for heating of a consumable based on a user profile of the user.

Hence, assessing the user aspect based on the modulated acoustic signal may allow adjusting function of the aerosol-generating device without burdening the user with additional actions for user identification.

The aspect may comprise the consumable aspect, wherein the information related to the aspect allows assessing a consumable inserted in the aerosol-generating device. The one or more reference signals comprise a reference signal of an authentic consumable, wherein the acoustic analysis system is configured to verify the consumable by comparing an acoustic signature of the consumable with the reference signal of an authentic consumable.

The consumable may be comprised in a plug of tobacco. The tobacco plug may comprise one or more of: powder, granules, pellets, shreds, spaghettis, strips or sheets containing one or more of: tobacco leaf, fragments of tobacco ribs, reconstituted tobacco, homogenized tobacco, extruded tobacco and expanded tobacco. Optionally, the tobacco plug may contain additional tobacco or non-tobacco volatile flavor compounds, to be released upon heating of the tobacco plug. Optionally, the tobacco plug may also contain capsules that, for example, include the additional tobacco or non-tobacco volatile flavor compounds. Such capsules may melt during heating of the tobacco plug. Alternatively, or in addition, such capsules may be crushed prior to, during, or after heating of the tobacco plug.

Where the tobacco plug comprises homogenized tobacco material, the homogenized tobacco material may be formed by agglomerating particulate tobacco. The homogenized tobacco material may be in the form of a sheet. The homogenized tobacco material may have an aerosol-former content of greater than 5 percent on a dry weight basis. The homogenized tobacco material may alternatively have an aerosol former content of between 5 percent and 30 percent by weight on a dry weight basis. Sheets of homogenized tobacco material may be formed by agglomerating particulate tobacco obtained by grinding or otherwise comminuting one or both of tobacco leaf lamina and tobacco leaf stems; alternatively, or in addition, sheets of homogenized tobacco material may comprise one or more of tobacco dust, tobacco fines and other particulate tobacco by-products formed during, for example, the treating, handling and shipping of tobacco. Sheets of homogenized tobacco material may comprise one or more intrinsic binders, that is tobacco endogenous binders, one or more extrinsic binders, that is tobacco exogenous binders, or a combination thereof to help agglomerate the particulate tobacco. Alternatively, or in addition, sheets of homogenized tobacco material may comprise other additives including, but not limited to, tobacco and non-tobacco fibers, aerosol-formers, humectants, plasticizers, flavourants, fillers, aqueous and non-aqueous solvents and combinations thereof. Sheets of homogenized tobacco material are preferably formed by a casting process of the type generally comprising casting a slurry comprising particulate tobacco and one or more binders onto a conveyor belt or other support surface, drying the cast slurry to form a sheet of homogenized tobacco material and removing the sheet of homogenized tobacco material from the support surface.

The consumable may be comprised in an aerosol-generating article. The aerosol-generating article may have a total length of between approximately 30 millimeters and approximately 100 millimeters. The aerosol-generating article may have an external diameter of between approximately 5 millimeters and approximately 13 millimeters.

The aerosol-generating article may comprise a mouthpiece positioned downstream of the tobacco plug. The mouthpiece may be located at a downstream end of the aerosol-generating article. The mouthpiece may be a cellulose acetate filter plug. Preferably, the mouthpiece is approximately 7 millimeters in length, but can have a length of between approximately 5 millimeters to approximately 10 millimeters.

The tobacco plug may have a length of approximately 10 millimeters. The tobacco plug may have a length of approximately 12 millimeters.

The diameter of the tobacco plug may be between approximately 5 millimeters and approximately 12 millimeters.

Preferably, the aerosol-generating article has a total length of between approximately 40 millimeters and approximately 50 millimeters. Preferably, the aerosol-generating article has a total length of approximately 45 millimeters. Preferably, the aerosol-generating article has an external diameter of approximately 7.2 millimeters.

Thereby, information related to the consumable aspect may allow verifying the consumable as an authentic consumable. Hence, the user is protected from inhaling potentially harmful aerosols from unsuitable or counterfeited consumables.

According to another aspect of the present invention, a method for capturing information related to an aspect associated with an aerosol-generating device is provided, the aspect comprising at least one of a user aspect and a consumable aspect. The method comprises, in response to a user activating a button on the aerosol-generating device, generating an acoustic signal, wherein the acoustic signal is modulated at least in part according to the aspect. The user activating the button comprises the user pressing the button. The method further comprises detecting the modulated acoustic signal by an acoustic sensor associated with the aerosol-generating device, thereby capturing the information related to the aspect, wherein the pressing of the button generates the acoustic signal from a mechanical motion of the button.

By detecting the modulated acoustic signal, information on physical aspects of the materials or the human body can be captured, so as to characterize the material or the human body without relying on digital markers prone to counterfeiting.

The method may further comprise comparing the detected acoustic signal with one or more reference signals for assessing the aspect, wherein the aspect comprises the user aspect, wherein the method further comprises performing the steps of generating the acoustic signal and detecting the modulated acoustic signal several times to record a bioacoustic profile for the user to be employed as the reference signal for authenticating the user for the assessing the user aspect.

Hence, the reference signal for authenticating the user can be captured with the aerosol-generating device without relying on additional dedicated hardware.

As employed in the present disclosure, the term user aspect relates to aspects relating to user of the aerosol generating device, for example his or her identity and age, so that according to the user aspect the user may for example authenticated using the captured information. As employed in the present disclosure, the term consumable aspect relates to aspects of a consumable inserted in the aerosol generating device, for example brand authenticity, or remaining quality of the consumable.

The invention is defined in the claims. However, below there is provided a non-exhaustive list of non-limiting examples. Any one or more of the features of these examples may be combined with any one or more features of another example, embodiment, or aspect described herein.
Figures 1A and 1B illustrate an aerosol generating device for which the present invention may be applied;
Figures 2A and 2B illustrate cross sections of the aerosol generating device of Figures 1A and 1B;
Figure 3 illustrates components of a system for capturing an aspect associated with an aerosol generating device;
Figure 4 illustrates a process flow diagram within the components of the system of Figure 3; and
Figure 5 illustrates a flow diagram of a method for capturing an aspect related to an aerosol generating device.

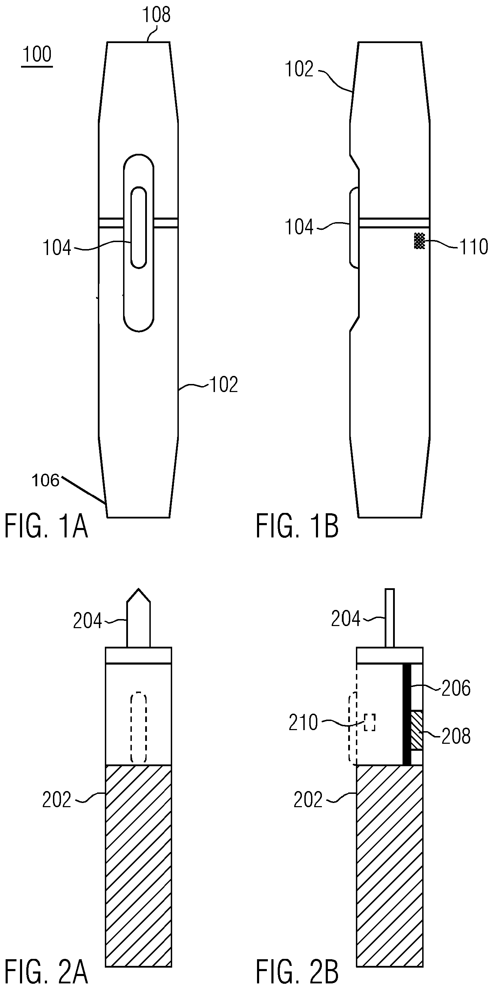

Figures 1A and 1B illustrate external views of aerosol generating device 100 having an associated user aspect or a consumable aspect whose information is captured by a system, such as system 30 described below. Aerosol generating device 100 has casing 102 and button 104 located on casing 102. Aerosol generating device 100 comprises mouthpiece 106 for providing aerosols to a user, and backend 108, typically having with connectors for recharging a battery of aerosol generating device 100.

Figure 1B illustrates a side view of aerosol generating device 100. In Figure 1B, bioacoustic signal input region 110 is indicated, where input of the modulated acoustic signal from a user's finger should advantageously be provided for capturing information related to the user aspect.

Pressing button 104 generates an acoustic signal from the mechanical motion of button 104. Additionally, button 104 may, in addition to powering on aerosol generating device 100, activate one or more vibrators that generate the acoustic signal. In embodiments, button 104 is a power button of the aerosol generating device. Alternatively or additionally, button 104 may be a button for initiating a heating process of the aerosol generating device.

The user is usually holding aerosol generating device 100 with two fingers such that he or she can activate button 104 with one of the fingers. The generated acoustic signal may be transmitted to the user's finger with which he or she has pressed button 104. The acoustic signal may then travel over the user's finger and hand to another finger located near bioacoustic signal input region 110, so that bioacoustic signal input region 110 receives an acoustic signal modulated by the unique material properties and unique anatomy of the user's hand.

Bioacoustic signal input region 110 may be located at a location that is ergonomically preferred by most users for holding the aerosol generating device 100 with one finger, for example the thumb, while pressing button 104 with another finger.

Figures 2A and 2B illustrate sections through aerosol-generating device 100. The lower portion of the body of aerosol generating device 100 is occupied by battery 202. The top of the inner body of aerosol generating device 100 carries heating blade 204 configured to heat a consumable to produce aerosols. As illustrated in Figure 2B, control electronics 206 is located near the back of the body of aerosol generating device 100. Further, acoustic sensor 208 is located close to bioacoustic signal input region 110. Aerosol generating device 100 may further comprise vibration speaker 210 configured to be activated when a user presses button 104 to generate the acoustic signal.

Further, the acoustic signal generated by the button press may comprise a first acoustic signal generated upon a press to the button and a second acoustic signal generated upon a release of the button. In other embodiments, a press to button 104 activates the signal capture system comprised in control electronics 206 and a release of the button generates the acoustic signal.

Button 104, vibration speaker 210, and acoustic sensor 208 are located such that the acoustic signal generated by button 104 or vibration speaker 210 is modulated according to the user aspect or according to the consumable aspect before being detected by acoustic sensor 208.

As shown in Figures 1A to 2B, bioacoustic signal input region 110 and acoustic sensor 208 may be located at a back face of aerosol generating device 100, while button 104 is located on a front face. In embodiments, bioacoustic signal input region 110 and acoustic sensor 208 may be located opposite to the button 104 around the circumference of aerosol generating device 100, such that the user's digits, for example one of the user's fingers and the user's thumb touch the aerosol-generating device at these locations when a user conveniently holds the aerosol-generating device.

The location opposite to the location of the button 104 may correspond to between 140 degrees and 220 degrees measured along the circumference of aerosol generating device 100.

In other embodiments, the location of acoustic sensor 208 is in a smart watch associated with aerosol generating device 100. In these embodiments, the acoustic signal generated in response to a press of button 104 is transmitted via the finger or thumb, which the user employs to press the button, over the hand to the wrist carrying the smart watch, and is hence modulated by the anatomy of the user's hand.

In embodiments for capturing information related to the consumable aspect, the locations of the button 104, vibration speaker 210, and acoustic sensor 208 may be chosen such that the device's reservoir for consumables acts as a variable modulator to the acoustic signal. The information related to the consumable aspect may allow verification of brand authenticity of the consumable or provide a time variable signal improving long-term authentication. In further embodiments, the information related to the consumable aspect may allow estimating a remaining supply of the consumable. For capturing information related to the consumable aspect, vibration speaker 210 and acoustic sensor 208 may be such that a path from vibration speaker 210 to acoustic sensor 208 traverses the reservoir holding the consumable.

In embodiments, the acoustic signal is generated by vibration speaker 210 as a series of acoustic signals with a range of frequencies. The range of frequencies may be selected based on considering a quality of bioacoustic transmission. Further, employing a range of frequencies allows improving the modulation pattern because response of tissue to acoustic signals differentially varies between tissues. Casing 102 may have perforations around the acoustic signal input region 110 to improve signal transmission to acoustic sensor 208.

According to an embodiment, the user aspect relates to the user currently holding the aerosol generating device, and the captured information may allow authenticating the current user. In response to identifying the current user from among registered users, aerosol generating device 100 may be configured to adjust a process for heating of a consumable based on a stored user profile of the user. In response to failing to identify the user from among the registered users, the aerosol generating device may be configured to be locked, such as locked for a predetermined time. In embodiments, the aerosol generating device may further be configured for sending a notification to a registered user via a third party device of an attempted unauthorized use of the aerosol generating device. Unlocking the aerosol generating device may require approval from the third party device, such as a smart phone, a personal computer or a smart device.

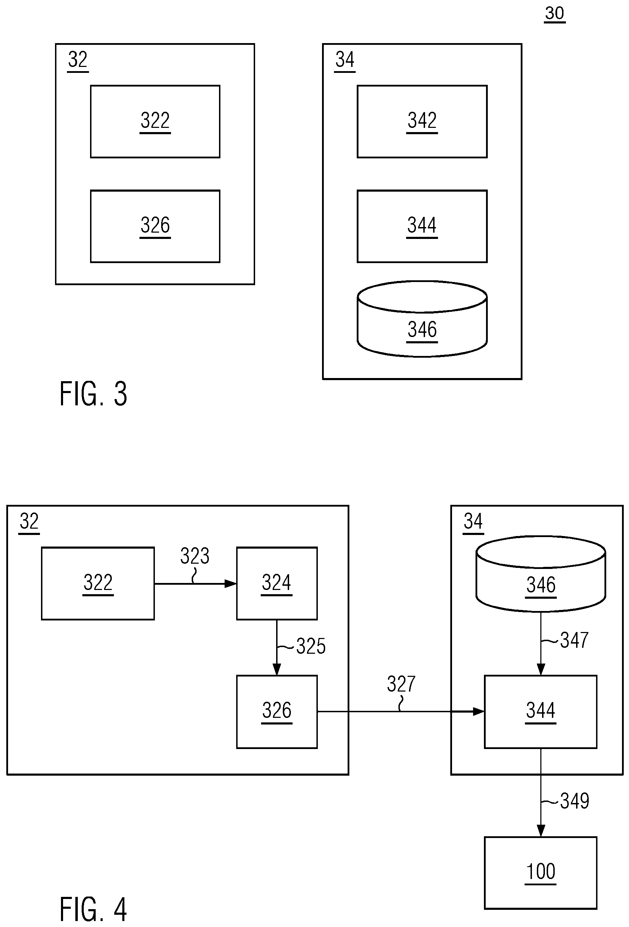

Figure 3 illustrates components of a system 30 for capturing information related to an aspect associated with an aerosol generating device. System 30 may be fully integrated in aerosol generating device 100. Alternatively, at least some components of system may be implemented on other devices that are connected to aerosol generating device 100 via a data connection. System 30 comprises signal capture system 32, which comprises acoustic actuators 322 and acoustic sensor 326. Acoustic actuators 322 are configured for generating an acoustic signal. As explained above with reference to Figures 1A to 2B, acoustic actuators 322 may comprise at least one of a power button, a heating button, or a vibration speaker of the aerosol-generating device.

According to embodiments, acoustic actuators 322 may comprise a vibration speaker with effective frequency range of 80 Hertz to 18 kiloHertz. In embodiments, frequencies selected may be between 0.5 kiloHertz to 10 kiloHertz. Acoustic sensor 322 may be a contact microphone, for example a BU27135 accelerometer with a sensitivity between -45 decibel ± 4.5 decibel.

System 32 may be activated when a user presses a button of the aerosol-generating device, employing a finger or thumb, upon which an acoustic signal is generated. The acoustic signal is transmitted through the aerosol-generating device and, according to embodiments, the human body, is modulated at least in part according to the aspect. The system comprises acoustic sensor 326 for detecting the modulated acoustic signal.

Components of signal capture system 32 may be comprised in the aerosol generating device. Alternatively, acoustic sensor 326 may be located remote from acoustic actuators 322, for example in a smart watch carried by a user.

In an embodiment, system 30 also comprises acoustic analysis system 34. Acoustic analysis system 34 comprises user interface 342, acoustic analysis algorithm 344 and reference database 346.

Acoustic analysis algorithm 344 is configured to obtain the modulated acoustic signal from acoustic sensor 326 and to compare the modulated acoustic signal with a reference signal from reference database 346 to assess the user aspect or the consumable aspect. Modulations of the acoustic signal are encoded in the harmonics of the signal, which may be analyzed by the acoustic analysis algorithm. Acoustic analysis algorithm 344 may utilize appropriate filtering techniques to remove environmental or other noise from the detected acoustic signal.

According to an embodiment, the user aspect relates to the user currently holding the aerosol generating device. Reference database 346 may accordingly store acoustic profiles of registered users of the aerosol generating device. In response to identifying the current user from among the registered users, a user profile that, for example, defines user settings for heating of a consumable may be transferred to the aerosol-generating device.

Acoustic analysis algorithm 344 may be executed locally on the aerosol generating device or remotely such as on a third party device or on a cloud server.

According to another aspect of the invention, reference database 346 may comprise a generic profile of an adult. The generic profile of an adult may be based on regional population average. An associated generic reference signal of an adult is influenced by soft tissue property, bone property and joint tension of a human which vary with age. As is well known, hand bone assessment allows inferring a development stage in adolescence because ossification processes increase density and material hardening of the bones with age of adolescence, so that the modulations of the acoustic signal vary with age. Further, fusion of the hypophysis and metaphysis influences the acoustic signal transmission. Hence, for a younger person, the acoustic signature will exhibit greater damping at higher frequency ranges due to reduced bone stiffness and fusion, which yields a feature to estimate an age of the user holding the aerosol generating device.

Hence, acoustic analysis algorithm 344 may compare features of the modulated acoustic signal with features of the generic profile of an adult to generate an estimated age of the user. The estimated age may be employed as a further measure safeguarding against unauthorized use.

Acoustic analysis system 34 may be executed at a same location where acoustic analysis system 34 is being performed, such as locally on the aerosol generating device. Alternatively, acoustic analysis system 34 may be executed at a remote location, such as a smart phone, a laptop computer, or a smart device, for example a smart watch.

Acoustic analysis system 34 may further comprise user interface 342 configured to guide the user to correctly use the acoustic analysis system 30. User interface 342 may be configured to provide haptic feedback to the user. For example, a vibration motor may configured the aerosol-generating device to generate vibrations when the user has not correctly placed their digits to allow assessment of the aspect. Hence, when acoustic analysis algorithm 344 determines that no assessment is possible from the captured acoustic signal, the user interface may be instructed to provide feedback accordingly. The feedback may also comprise visual feedback, for example by a light-emitting diode indicator.

Further, user interface 342 may communicate to the user when an error in authentication has occurred, and when the bioacoustic signature of the user has been accepted or rejected. In embodiments, user interface 342 may be configured to allow the user to try authentication for a predetermined number of times. After failing authentication in each of the predetermined number of times, mitigation steps may be taken to prevent unauthorized use, as explained above.

System 30 may be configured to assess a consumable inserted in aerosol generating device 100. An acoustic actuator of acoustic activators 322 may be located such that a path from the acoustic actuator to acoustic sensor 110 traverses the consumable, for example through an e-liquid chamber, heated tobacco product or other consumable, of the aerosol generating device 100. After the acoustic signal has been transmitted through the consumable, modulations picked up by the acoustic signal during the transmission through the consumable may allow characterizing the consumable.

Reference database 346 may comprise reference signals of authentic consumables provided by a manufacturer of the consumable. In alternative embodiments, the predetermined locations of the acoustic activators 322 and acoustic sensor 326 may be such that reflections of the modulated acoustic signal are captured for assessing the consumable. The acoustic signal is generated, as explained above, in response to the user pressing a button of the aerosol generating device. In addition to authenticating the consumable, based on analysis of the received acoustic signal, acoustic analysis algorithm 344 may also infer a remaining quantity of the consumable. Analysis of the remaining quantity of the consumable may be based on comparing the captured acoustic signal with a reference signal of no remaining quantity of the consumable. Alternatively, a consistent time variation in the signal obtained from analyzing the captured acoustic signal at each subsequent use, may be employed to estimate the remaining quantity of the consumable.

User interface 342 may communicate to the user acceptance or rejection of an acoustic signature of the consumable. Additionally or alternatively, user interface 342 may also communicate to the user a low quantity of remaining consumable. If acoustic analysis algorithm 344 estimates low level of remaining consumable, user interface 342 may inform the user of the low level of the consumable and may inquire the user whether they would like to order more of the consumable. Under prior authorization of the user, an order for replenishment of the consumable may be automatically placed.

Figure 4 illustrates signal and data flow in system 30. As illustrated, acoustic actuators 322, in response to a user button press, generate acoustic signal 323, which is transmitted to a finger, transmitted up one finger and down another finger 324 to yield a modulated acoustic signal 325, which includes user-specific modulations. Modulated acoustic signal 325 is captured at acoustic sensor 326. Captured signal data 327 of the acoustic signal are transferred from acoustic sensor 324 to acoustic analysis algorithm 344, which is executed at a potentially in a location remote from signal capture step system 32. Acoustic analysis algorithm 344 retrieves reference signals 347 from reference database 346 to provide an authentication decision 349 to aerosol generating device 100.

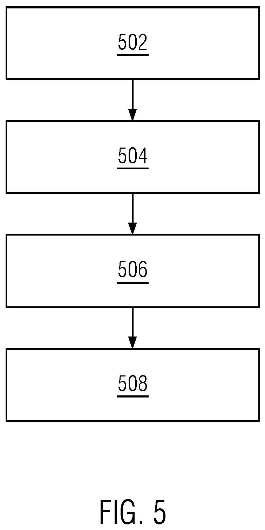

Figure 5 illustrates a flowchart of a method 500 for capturing information related to an aspect associated with an aerosol generating device. Method 500 comprises generating one or more acoustic signals in response to a user activating a button on the aerosol generating device. The signal passes through the user's hand, and, alternatively or additionally, through a reservoir holding consumables. Method 500 further comprises step 504 of capturing the modulated signal by an acoustic sensor associated with the aerosol generating device to capture the information related to the aspect. As explained above, method 500 may comprise step 506 of processing the captured acoustic signal by an acoustic analysis algorithm, including comparing the detected acoustic signal to a reference to assess the user aspect. Additionally, or alternatively, the detected acoustic signal may be compared to a reference consumable signal to assess the consumable aspect. When assessing the user aspect comprises authenticating the user, method 500 may comprise repeating steps 502 to 506 for a predetermined number of times before for example locking the device from use when the user could not be authenticated.

Method 500 may comprise guiding the user to correctly place their digit on the aerosol generating device by optic or visual feedback. Step 502 may comprise generating the acoustic signal upon a press of the button and a second acoustic signal upon a release of the button, or generating the acoustic signal by activating a vibration speaker to generate the acoustic signal at a specific frequency or generating a sequence of acoustic signals with a range of frequencies (or generating the acoustic signal upon a press of the button and a second acoustic signal upon a release of the button and generating the acoustic signal by activating the vibration speaker).

Method 500 comprises assessing the user aspect or the consumable aspect. As explained above, this may comprise authenticating a user, estimating an age of the user or verifying authenticity of a consumable inserted in the aerosol generating device.

Advantageously, the present invention also allows performing steps 502 and 504 to capture the reference signal for the user. Accordingly, when first using the aerosol generating device, user interface 242 may guide the user to repeatedly perform steps 502 and 504 to record a bioacoustic profile for the user to be employed as a reference signal for the user for later authenticating the user.

For the purpose of the present description and of the appended claims, except where otherwise indicated, all numbers expressing amounts, quantities, percentages, and so forth, are to be understood as being modified in all instances by the term "about". Also, all ranges include the maximum and minimum points disclosed and include any intermediate ranges therein, which may or may not be specifically enumerated herein. In this context, therefore, a number A is understood as A ± 10 % of A. Within this context, a number A may be considered to include numerical values that are within general standard error for the measurement of the property that the number A modifies. The number A, in some instances as used in the appended claims, may deviate by the percentages enumerated above provided that the amount by which A deviates does not materially affect the basic and novel characteristic(s) of the claimed invention. Also, all ranges include the maximum and minimum points disclosed and include any intermediate ranges therein, which may or may not be specifically enumerated herein.

## Claims

1. A system for capturing information related to an aspect associated with an aerosol-generating device (100), the aspect comprising at least one of a user aspect and a consumable aspect, the system comprising:
a signal capture system (32) comprising:
one or more acoustic actuators (322) configured for generating an acoustic signal, wherein the one or more acoustic actuators (322) comprise a button (104), and
an acoustic sensor (326),
wherein the one or more acoustic actuators (322) and the acoustic sensor (326) are located at predetermined locations with respect to the aerosol-generating device (100) such that an acoustic signal generated by pressing the button (104) is modulated at least in part according to the aspect before being detected by the acoustic sensor (326), thereby capturing the information related to the aspect,
**characterized in that**
the pressing of the button (104) generates the acoustic signal from a mechanical motion of the button (104).

2. The system of claim 1, wherein the button is an acoustic actuator of the one or more acoustic actuators (322).

3. The system of claim 1 or claim 2, wherein the predetermined locations correspond to points of contact of a user's digits on the aerosol-generating device (100) when the user is holding the aerosol-generating device.

4. The system of claim 3, wherein the acoustic signal generated by the button (104) comprises a first acoustic signal generated upon a press of the button (104) and a second acoustic signal generated upon a release of the button (104).

5. The system of claim 3, wherein the aerosol-generating device (100) is configured such that a press of the button (104) activates the signal capture system, and a release of the button (104) generates the acoustic signal.

6. The system of one of one of claims 1 to 5, further comprising a smart watch, wherein the smart watch comprises the acoustic sensor (326).

7. The system of one of claims 1 to 6, wherein the one or more acoustic actuators (322) comprise a vibration speaker (210), wherein the acoustic signal comprises a series of acoustic signals generated by the vibration speaker (210).

8. The system of one of claims 1 to 7, wherein a casing of the aerosol-generating device (100) comprises perforations at the predetermined location of the acoustic sensor (208; 326).

9. The system of one of claims 1 to 8, further comprising an acoustic analysis system (34) configured for assessing the aspect on basis of the detected acoustic signal, wherein the acoustic analysis system (34) is configured for assessing the aspect by comparing the detected acoustic signal with one or more reference signals.

10. The system of one of claims 1 to 9, wherein the aspect comprises the user aspect, wherein the information related to the user aspect allows assessing a user of the aerosol-generating device, wherein the one or more reference signals comprise bioacoustic profiles of registered users of the aerosol-generating device, wherein the acoustic analysis system is configured for performing an identification of the user from among the registered users.

11. The system of claim 10, wherein the aerosol-generating device (100) is configured to, in response to identifying the user, adjust a process for heating of a consumable based on a user profile of the user.

12. The system of one of claims 1 to 11, wherein the aspect comprises the consumable aspect, wherein the information related to the aspect allows assessing a consumable inserted in the aerosol-generating device (100).

13. The system of claim 12, wherein the one or more reference signals comprise a reference signal of an authentic consumable, wherein the acoustic analysis system (34) is configured to verify the consumable by comparing an acoustic signature of the consumable with the reference signal of an authentic consumable.

14. A method for capturing information related to an aspect associated with an aerosol-generating device, the aspect comprising at least one of a user aspect and a consumable aspect, the method comprising:
in response to a user activating a button on the aerosol-generating device, generating (502) an acoustic signal, wherein the acoustic signal is modulated at least in part according to the aspect, wherein the user activating the button comprises the user pressing the button;
detecting (504) the modulated acoustic signal by an acoustic sensor associated with the aerosol-generating device, thereby capturing the information related to the aspect,
**characterized in that**
the pressing of the button generates the acoustic signal from a mechanical motion of the button.

15. The method of claim 14, wherein the button is employed as an acoustic actuator.

16. The method of claim 14, wherein the aspect comprises the user aspect, the method further comprising performing the steps of generating (502) the acoustic signal and detecting (504) the modulated acoustic signal several times to record a bioacoustic profile for the user, wherein the method further comprises, at a later time, comparing (506) the detected acoustic signal with the recorded bioacoustic profile.

## Patentansprüche

1. System zum Erfassen von Informationen, die sich auf einen Aspekt beziehen, der einer Aerosolerzeugungsvorrichtung (100) zugewiesen ist, wobei der Aspekt wenigstens einen von einem Benutzeraspekt und einem Verbrauchsartikelaspekt umfasst, das System umfassend:
ein Signalerfassungssystem (32), umfassend:
einen oder mehrere akustische Aktoren (322), die für ein Erzeugen eines akustischen Signals eingerichtet sind, wobei der eine oder die mehrere akustischen Aktoren (322) eine Taste (104) aufweisen, und
einen akustischen Sensor (326),
wobei sich der eine oder die mehreren akustischen Aktoren (322) und der akustische Sensor (326) an vorbestimmten Stellen in Bezug auf die Aerosolerzeugungsvorrichtung (100) befinden, sodass ein durch Drücken der Taste (104) erzeugtes akustisches Signal wenigstens teilweise gemäß dem Aspekt moduliert wird, bevor es von dem akustischen Sensor (326) detektiert wird, wodurch die Informationen bezüglich des Aspekts erfasst werden,
**dadurch gekennzeichnet, dass**
das Drücken der Taste (104) das akustische Signal durch eine mechanische Bewegung der Taste (104) erzeugt.

2. System nach Anspruch 1, wobei die Taste ein akustischer Aktor des wenigstens einen akustischen Aktors (322) ist.

3. System nach Anspruch 1 oder Anspruch 2, wobei die vorbestimmten Stellen Kontaktpunkten der Finger eines Benutzers auf der Aerosolerzeugungsvorrichtung (100) entsprechen, wenn der Benutzer die Aerosolerzeugungsvorrichtung hält.

4. System nach Anspruch 3, wobei das durch die Taste (104) erzeugte akustische Signal ein erstes akustisches Signal, das bei einem Drücken der Taste (104) erzeugt wird, und ein zweites akustisches Signal, das bei einem Loslassen der Taste (104) erzeugt wird, umfasst.

5. System nach Anspruch 3, wobei die Aerosolerzeugungsvorrichtung (100) derart ausgelegt ist, dass ein Drücken der Taste (104) das Signalerfassungssystem aktiviert und ein Loslassen der Taste (104) das akustische Signal erzeugt.

6. System nach einem der Ansprüche 1 bis 5, ferner umfassend eine Smartwatch, wobei die Smartwatch den akustischen Sensor (326) umfasst.

7. System nach einem der Ansprüche 1 bis 6, wobei der eine oder die mehrere akustischen Aktoren (322) einen Vibrationslautsprecher (210) aufweisen, wobei das akustische Signal eine Reihe von akustischen Signalen umfasst, die von dem Vibrationslautsprecher (210) erzeugt werden.

8. System nach einem der Ansprüche 1 bis 7, wobei ein Gehäuse der Aerosolerzeugungsvorrichtung (100) Perforationen an der vorbestimmten Stelle des akustischen Sensors (208; 326) aufweist.

9. System nach einem der Ansprüche 1 bis 8, ferner aufweisend ein akustisches Analysesystem (34), das dazu eingerichtet ist, den Aspekt basierend auf dem detektierten akustischen Signal zu bewerten, wobei das akustische Analysesystem (34) dazu eingerichtet ist, den Aspekt durch Vergleichen des detektierten akustischen Signals mit einem oder mehreren Referenzsignalen zu bewerten.

10. System nach einem der Ansprüche 1 bis 9, wobei der Aspekt den Benutzeraspekt umfasst, wobei die Informationen bezüglich des Benutzeraspekts eine Bewertung eines Benutzers der Aerosolerzeugungsvorrichtung ermöglichen, wobei das eine oder die mehreren Referenzsignale bioakustische Profile registrierter Benutzer der Aerosolerzeugungsvorrichtung umfassen, wobei das akustische Analysesystem dazu eingerichtet ist, eine Identifizierung des Benutzers unter den registrierten Benutzern durchzuführen.

11. System nach Anspruch 10, wobei die Aerosolerzeugungsvorrichtung (100) dazu ausgelegt ist, in Reaktion auf das Identifizieren des Benutzers einen Prozess für das Erwärmen eines Verbrauchsartikels basierend auf einem Benutzerprofil des Benutzers anzupassen.

12. System nach einem der Ansprüche 1 bis 11, wobei der Aspekt den Verbrauchsartikelaspekt umfasst, wobei die Informationen bezüglich des Aspekts eine Bewertung eines in die Aerosolerzeugungsvorrichtung (100) eingesetzten Verbrauchsartikels ermöglichen.

13. System nach Anspruch 12, wobei das eine oder die mehrere Referenzsignale ein Referenzsignal eines authentischen Verbrauchsartikels umfassen, wobei das akustische Analysesystem (34) dazu eingerichtet ist, den Verbrauchsartikel durch Vergleichen einer akustischen Signatur des Verbrauchsartikels mit dem Referenzsignal eines authentischen Verbrauchsartikels zu verifizieren.

14. Verfahren zum Erfassen von Informationen, die sich auf einen Aspekt beziehen, der einer Aerosolerzeugungsvorrichtung zugewiesen ist, wobei der Aspekt wenigstens einen von einem Benutzeraspekt und einem Verbrauchsartikelaspekt umfasst, das Verfahren umfassend:
in Reaktion auf das Betätigen einer Taste an der Aerosolerzeugungsvorrichtung durch einen Benutzer, Erzeugen (502) eines akustischen Signals, wobei das akustische Signal wenigstens teilweise gemäß dem Aspekt moduliert wird, wobei das Betätigen der Taste durch den Benutzer das Drücken der Taste durch den Benutzer umfasst;
Detektieren (504) des modulierten akustischen Signals durch einen der Aerosolerzeugungsvorrichtung zugewiesenen akustischen Sensor, wodurch die Informationen bezüglich des Aspekts erfasst werden,
**dadurch gekennzeichnet, dass**
das Drücken der Taste das akustische Signal durch eine mechanische Bewegung der Taste erzeugt.

15. Verfahren nach Anspruch 14, wobei die Taste als akustischer Aktor verwendet wird.

16. Verfahren nach Anspruch 14, wobei der Aspekt den Benutzeraspekt umfasst, wobei das Verfahren ferner das Ausführen der Schritte des Erzeugens (502) des akustischen Signals und des mehrmaligen Detektierens (504) des modulierten akustischen Signals umfasst, um ein bioakustisches Profil für den Benutzer aufzuzeichnen, wobei das Verfahren ferner das spätere Vergleichen (506) des detektierten akustischen Signals mit dem aufgezeichneten bioakustischen Profil umfasst.

## Revendications

1. Système destiné à capturer des informations relatives à un aspect associé à un dispositif de génération d'aérosol (100), l'aspect comprenant au moins l'un d'un aspect utilisateur et d'un aspect consommable, le système comprenant :
un système de capture de signal (32) comprenant :
un ou plusieurs actionneurs acoustiques (322) configurés pour générer un signal acoustique, dans lequel un ou plusieurs actionneurs acoustiques (322) comprennent un bouton (104), et
un capteur acoustique (326),
dans lequel le ou les actionneurs acoustiques (322) et le capteur acoustique (326) sont situés à des emplacements prédéterminés par rapport au dispositif de génération d'aérosol (100) de telle sorte qu'un signal acoustique généré en appuyant sur le bouton (104) est modulé au moins en partie selon l'aspect avant d'être détecté par le capteur acoustique (326), captant ainsi les informations liées à l'aspect,
**caractérisé en ce que**
la pression sur le bouton (104) génère le signal acoustique à partir d'un mouvement mécanique du bouton (104).

2. Système selon la revendication 1, dans lequel le bouton est un actionneur acoustique du ou des actionneurs acoustiques (322).

3. Système selon la revendication 1 ou la revendication 2, dans lequel les emplacements prédéterminés correspondent à des points de contact des doigts d'un utilisateur sur le dispositif de génération d'aérosol (100) lorsque l'utilisateur tient le dispositif de génération d'aérosol.

4. Système selon la revendication 3, dans lequel le signal acoustique généré par le bouton (104) comprend un premier signal acoustique généré lors d'une pression du bouton (104) et un deuxième signal acoustique généré lors d'un relâchement du bouton (104).

5. Système selon la revendication 3, dans lequel le dispositif de génération d'aérosol (100) est configuré de telle sorte qu'une pression du bouton (104) active le système de capture de signal, et qu'un relâchement du bouton (104) génère le signal acoustique.

6. Système selon l'une des revendications 1 à 5, comprenant en outre une montre intelligente, dans laquelle la montre intelligente comprend le capteur acoustique (326).

7. Système selon l'une des revendications 1 à 6, dans lequel le ou les actionneurs acoustiques (322) comprennent un haut-parleur à vibration (210), dans lequel le signal acoustique comprend une série de signaux acoustiques générés par le haut-parleur à vibration (210).

8. Système selon l'une des revendications 1 à 7, dans lequel un boîtier du dispositif de génération d'aérosol (100) comprend des perforations à l'emplacement prédéterminé du capteur acoustique (208 ; 326).

9. Système selon l'une des revendications 1 à 8, comprenant en outre un système d'analyse acoustique (34) configuré pour évaluer l'aspect sur la base du signal acoustique détecté, dans lequel le système d'analyse acoustique (34) est configuré pour évaluer l'aspect en comparant le signal acoustique détecté à un ou plusieurs signaux de référence.

10. Système selon l'une des revendications 1 à 9, dans lequel l'aspect comprend l'aspect utilisateur, dans lequel les informations relatives à l'aspect utilisateur permettent d'évaluer un utilisateur du dispositif de génération d'aérosol, dans lequel le ou les signaux de référence comprennent des profils bioacoustiques d'utilisateurs enregistrés du dispositif de génération d'aérosol, dans lequel le système d'analyse acoustique est configuré pour effectuer une identification de l'utilisateur parmi les utilisateurs enregistrés.

11. Système selon la revendication 10, dans lequel le dispositif de génération d'aérosol (100) est configuré pour, en réponse à l'identification de l'utilisateur, ajuster un processus de chauffage d'un consommable sur la base d'un profil d'utilisateur de l'utilisateur.

12. Système selon l'une des revendications 1 à 11, dans lequel l'aspect comprend l'aspect consommable, dans lequel les informations relatives à l'aspect permettent d'évaluer un consommable inséré dans le dispositif de génération d'aérosol (100).

13. Système selon la revendication 12, dans lequel le ou les signaux de référence comprennent un signal de référence d'un consommable authentique, dans lequel le système d'analyse acoustique (34) est configuré pour vérifier le consommable en comparant une signature acoustique du consommable avec le signal de référence d'un consommable authentique.

14. Procédé pour capturer des informations relatives à un aspect associé à un dispositif de génération d'aérosol, l'aspect comprenant au moins l'un d'un aspect utilisateur et d'un aspect consommable, le procédé comprenant :
en réponse à l'activation, par un utilisateur, d'un bouton sur le dispositif de génération d'aérosol, la génération (502) d'un signal acoustique, dans lequel le signal acoustique est modulé au moins en partie en fonction de l'aspect, dans lequel l'activation du bouton par l'utilisateur comprend l'appui de l'utilisateur sur le bouton ;
la détection (504) du signal acoustique modulé par un capteur acoustique associé au dispositif de génération d'aérosol, capturant ainsi les informations relatives à l'aspect,
**caractérisé en ce que**
la pression sur le bouton génère le signal acoustique à partir d'un mouvement mécanique du bouton.

15. Procédé selon la revendication 14, dans lequel le bouton est utilisé comme actionneur acoustique.

16. Procédé selon la revendication 14, dans lequel l'aspect comprend l'aspect utilisateur, le procédé comprenant en outre l'exécution des étapes consistant à générer (502) le signal acoustique et à détecter (504) le signal acoustique modulé plusieurs fois afin d'enregistrer un profil bioacoustique de l'utilisateur, dans lequel le procédé comprend en outre, à un moment ultérieur, la comparaison (506) du signal acoustique détecté avec le profil bioacoustique enregistré.
